(19) Europäisches Patentamt
European Patent Office
Office européen des brevets

(11) Veröffentlichungsnummer: **0 226 543 B1**

(12) **EUROPÄISCHE PATENTSCHRIFT**

(45) Veröffentlichungstag der Patentschrift: **29.09.93**

(51) Int. Cl.$^5$: **C08G 59/06**, C08L 63/00

(21) Anmeldenummer: **86810575.0**

(22) Anmeldetag: **08.12.86**

Die Akte enthält technische Angaben, die nach dem Eingang der Anmeldung eingereicht wurden und die nicht in dieser Patentschrift enthalten sind.

# (54) Verbessertes Verfahren zur Herstellung von Glycidylverbindungen.

(30) Priorität: **13.12.85 CH 5316/85**

(43) Veröffentlichungstag der Anmeldung:
**24.06.87 Patentblatt 87/26**

(45) Bekanntmachung des Hinweises auf die Patenterteilung:
**29.09.93 Patentblatt 93/39**

(84) Benannte Vertragsstaaten:
**CH DE ES FR GB IT LI NL**

(56) Entgegenhaltungen:
**EP-A- 96 006**
**DD-B- 153 882**
**US-A- 3 033 821**
**EP-A- 0 013 532**
**FR-A- 1 215 551**
**US-A- 3 206 482**

(73) Patentinhaber: **CIBA-GEIGY AG**
**Klybeckstrasse 141**
**CH-4002 Basel(CH)**

(72) Erfinder: **Monnier, Charles E., Dr.**
**Ch. du Verger 16**
**CH-1752 Villars-sur-Glâne(CH)**
Erfinder: **Stockinger, Friedrich**
**Au Fernotz**
**CH-1784 Courtepin(CH)**

**EP 0 226 543 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Glycidylverbindungen und die Verwendung dieser Produkte auf den Gebieten des Oberflächenschutzes, der Elektrotechnik und Elektronik, der Laminierverfahren und im Bauwesen.

Epoxidharze werden seit langem in der Elektronikindustrie verwendet. In den letzten Jahren sind die Anforderungen an die Reinheit dieser Harze ständig gewachsen. Insbesondere können geringe Mengen an ionischem oder hydrolysierbarem Halogen störend wirken. Auch werden zunehmend Harze mit einem möglichst hohen Epoxidierungsgrad verlangt. In der Regel werden Epoxidharze durch Umsetzen von Verbindungen mit reaktiven Wasserstoffatomen, vorzugsweise Phenolen, mit Epichlorhydrin hergestellt. Die Reaktion erfolgt im allgemeinen in wässrigem Medium unter Verwendung einer basischen Verbindung, bevorzugt wässriger Natronlauge.

Es gibt Vorschläge, die Glycidylierungsreaktion in organischen Lösungsmitteln durchzuführen. Häufig wird dabei jedoch mit wässrigem Alkalihydroxid gearbeitet, so dass keine wasserfreien Reaktionsbedingungen vorliegen oder das Phenol wird in Form seines Alkalisalzes eingesetzt, so dass eine zusätzliche Verfahrensstufe notwendig wird. Beispiele für solche Reaktionsführungen sind in der DE-AS 1,081,666 (Umsetzung des Alkalisalzes eines Phenols mit Halogenhydrin unter wasserfreien Bedingungen in einem organischen Lösungsmittel, beispielsweise einem Alkohol oder Keton) oder in der JP-OS 60-31,517 bzw. der DD-PS 153,882 (Umsetzung eines Polyphenols mit Epichlorhydrin in aprotonischen polaren Lösungsmitteln in Gegenwart von wässriger Natronlauge) beschrieben.

Auch ist aus der DE-OS 2,533,505 ein zweistufiges Verfahren zur Herstellung von Glycidylverbindungen bekannt, worin ein Polyphenol mit Epichlorhydrin zunächst in Gegenwart eines Alkylenphosphorans umgesetzt wird und anschliessend durch Zugabe von u.a. festem Alkalihydroxid aus reagieren gelassen wird.

Ferner beschreibt die US-PS 4,518,762 ein verbessertes Verfahren zur Herstellung von Glycidylethern von Novolakharzen, bei dem die Reaktanden in Gegenwart einer geringen Menge Wasser und in einem Alkohol als Lösungsmittel umgesetzt werden. Als basische Verbindung verwendet man festes Alkalihydroxid, das im Laufe der Reaktion portionsweise zugesetzt wird. Die Verwendung von Kaliumcarbonat bei Glycidylierungsreaktionen wird von D.E. McClure et al. in JACS 101, 3666 (1979) beschrieben. Als Lösungsmittel bei der Reaktion dienen Aceton oder Dichlormethan.

Aus der DE-OS 2,205,097 ist ein Verfahren zur Vorverlängerung von Glycidylverbindungen bekannt, bei dem niedermolekulare Epoxidverbindungen mit zweiwertigen Phenolen in einem inerten Lösungsmittel umgesetzt werden. Als Katalysatoren werden Alkalihydroxide, quaternäre Ammonium- oder Phosphoniumssalze oder tertiäre Phosphine benötigt. Als Lösungsmittel werden insbesondere chlorierte Kohlenwasserstoffe, Ketone oder Diethylenglykol(ether) vorgeschlagen. Die Reaktionstemperatur beträgt bei diesem Verfahren 100-195 °C.

Schliesslich ist in Ausführungsbeispiel 2 der EP-A 96,006 ein Verfahren beschrieben, bei dem ein Phenol mit einem Ueberschuss an Epichlorhydrin in einem Aceton/Hexan Gemisch als Lösungsmittel in Gegenwart von pulverisiertem Kaliumcarbonat umgesetzt wird. Die Reaktion erfolgt unter Schutzgas bei der Siedetemperatur des Reaktionsgemisches. Die Reaktionsdauer beträgt 42 Stunden.

Es wurde jetzt eine Verfahrensführung gefunden, bei der die Glycidylierungsreaktion (vergl. Schema) unter sehr milden Bedingungen rasch und und mit hohen Ausbeuten abläuft. Dabei entstehen Produkte mit einem niedrigen Gehalt an hydrolysierbarem und ionischem Halogen und mit einem hohen Gehalt an Epoxidgruppen.

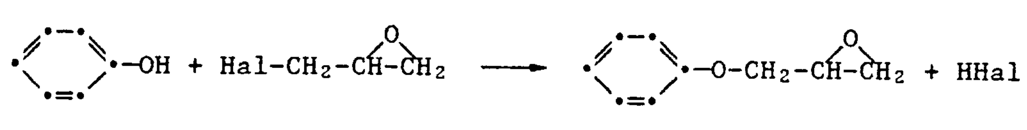

Die vorliegende Erfindung betrifft ein Verfahren zur Herstellung von Glycidylverbindungen durch Umsetzung einer mindestens eine phenolische Gruppe enthaltenden Verbindung mit mindestens der aequivalenten Menge, bezogen auf die phenolische Gruppe, eines Halogenhydrins in einem im wesentlichen wasserfreien, aprotonischen Lösungsmittel in Gegenwart einer festen, im wesentlichen wasserfreien, basischen Verbindung, dadurch gekennzeichnet, dass a) die Umsetzung bei einer Temperatur zwischen 40 und 80 °C in Gegenwart von Alkalicarbonat erfolgt, wobei ein aprotonisches, dipolares Lösungsmittel mit einer statischen relativen Dielektrizitätskonstante von mehr als 25 (bei 25 °C) und einem permanenten elektrischen Dipolmoment von mehr als 2,5 D eingesetzt wird, das zusätzlich durch eine Uebergangsenergie der solvatochromen Absorptionsbande des gelösten N-(3,5-Diphenyl-4-hydroxyphenyl)-2,4,6-triphenylpyridiniumperchlorates zwischen 168 und 197,4 kJ/Mol (bei 25 ° C) gekennzeichnet ist.

Zu den Verbindungen mit mindestens einer phenolischen Hydroxylgruppe, die im Rahmen dieser Erfindung eingesetzt werden können, zählen Substanzen mit einem oder mehreren Phenolkernen, wobei diese Kerne gegebenenfalls auch mehrere Hydroxylgruppen, bevorzugt jedoch eine Hydroxylgruppe, tragen können. Die Phenolkerne können unsubstituiert sein oder noch zusätzliche Substituenten aufweisen, beispielsweise ein bis vier Halogenatome, bevorzugt Chlor- oder Bromatome, Alkylgruppen, bevorzugt Methyl, Alkenylgruppen, bevorzugt Ethenyl oder Allyl, Phenyl- oder Benzylgruppen.

Beispiele für bevorzugt eingesetzte phenolische Komponenten sind Phenol, o-, m- oder p-Kresol, 2-Allylphenol, 2,6-Diallylphenol, 2,4,6-Triallylphenol, Bis-(Hydroxyphenyl)-methan (Bisphenol F; Isomerengemisch aus o,o'-, o,p'- und p,p'-Verbindungen), 1,1-Bis-(4-hydroxyphenyl)-ethan, 2,2-Bis-(4-hydroxyphenyl)-propan (Bisphenol A), Tetrabrom-Bisphenol A, Tetrachlor-Bisphenol A, 1,1-Bis-(4-hydroxyphenyl)-1-phenylethan, 1,1',2,2'-Tetrakis-(4-hydroxyphenyl)-ethan, Resorcin, Hydrochinon oder Polyphenole, die sich von der Kondensation von einkernigen Phenolen mit Aldehyden oder Ketonen, insbesondere mit Formaldehyd, ableiten (Novolake). Beispiele dafür sind Phenolnovolake, o-Kresolnovolake, p-Kresolnovolake oder Resorcinnovolake. Besonders bevorzugt verwendet man als phenolische Komponente Bisphenol A, Tetrabrom-Bisphenol A, Phenol-Novolake oder Kresolnovolake, insbesondere o-Kresolnovolake. Es können auch Gemische dieser Verbindungen verwendet werden. Die eingesetzten Novolake besitzen vorzugsweise Molekulargewichte von 300 bis 1000 (Zahlenmittel), ganz besonders jedoch Molekulargewichte von 400 bis 700.

Als Halogenhydrin eignen sich zum Beispiel Epichlorhydrin, ß-Methylepichlorhydrin,Epibromhydrin, ß-Methylepibromhydrin, Glycerindichlorhydrin oder Gemische dieser Verbindungen. Vorzugsweise verwendet man Epichlorhydrin.

Die Auswahl eines geeigneten Lösungsmittels spielt eine wesentliche Rolle bei der Durchführung des erfindungsgemässen Verfahrens. So eignet sich nicht jedes aprotonische, dipolare, Lösungsmittel, um die Reaktion in der gewünschten Geschwindigkeit ablaufen zu lassen. Ferner sind die Begriffe 'aprotonisches, dipolares Lösungsmittel' nicht eindeutig definiert (vergl. Chr. Reichardt in "Lösungsmittel-Effekte in der organischen Chemie"; Kap. 3.4; Verlag Chemie, 1973).

Eine Möglichkeit zur Charakterisierung der Polarität eines Lösungsmittels besteht darin, dass man die Uebergangsenergie der solvatochromen Absorptionsbande eines gelösten Pyridinium-N-Phenol-Betains bestimmt. Diese Methode ist von K. Dimroth et al. in Annalen der Chemie 661, 1 (1963) beschrieben.

Beispiele für aprotonische, dipolare Lösungsmittel sind 4-Methyl-1,3-dioxo-2-on (Propylencarbonat), Acetonitril, Dimethylsulfoxid, Tetramethylensulfon (Sulfolan), Dimethylformamid, Dimethylacetamid, 1-Methyl-2-pyrrolidon, Nitrobenzol oder Hexamethyl-phosphorsäuretriamid.

Besonders bevorzugt werden aprotonische, dipolare Lösungsmittel, wie oben definiert, mit einer statischen relativen Dielektrizitätskonstante von mehr als 30. Beispiele für solche Lösungsmittel sind 4-Methyl-1,3-dioxo-2-on, Acetonitril, Dimethylsulfoxid, Tetramethylensulfon, Dimethylformamid, Dimethylacetamid und 1-Methyl-2-pyrrolidon.

Bevorzugt verwendet man Dimethylsulfoxid und/oder Dimethylformamid, und ganz besonders bevorzugt Dimethylsulfoxid. Es können auch Lösungsmittelgemische eingesetzt werden, sofern sie unter die oben gegebene Definition fallen.

Das Lösungsmittel sollte im wesentlichen wasserfrei sein; vorzugsweise liegt der Wassergehalt des eingesetzten Lösungsmittels unterhalb von 1 Gew.%, besonders bevorzugt unterhalb 0,5 Gew.%.

Die basische Verbindung beim erfindungsgemässen Verfahren sollte ebenfalls im wesentlichen wasserfrei sein und in fester Form vorliegen. Sie kann gekörnt sein oder in Pulverform vorliegen. Vorzugsweise verwendet man einen pulverisierten Stoff.

Die Gesamtmenge der basischen Verbindung kann zu Beginn der Reaktion im Lösungsmittel gelöst oder teilweise suspendiert vorgelegt werden. Man kann die basische Verbindung aber auch während der Reaktion portionsweise oder kontinuierlich zuführen.

Als Alkalicarbonat verwendet man beispielsweise Natriumcarbonat, Kaliumcarbonat oder Cäsiumcarbonat. Bevorzugt verwendet man Kaliumcarbonat oder Cäsiumcarbonat; besonders bevorzugt setzt man Kaliumcarbonat ein.

Es können auch Gemische der einzelnen basischen Verbindungen verwendet werden, sofern diese unter die Definition der Verfahrensvariante fallen. Das erfindungsgemässe Verfahren kann gegebenenfalls unter Schutzgas, beispielsweise Stickstoff oder Argon, durchgeführt werden.

Die Reaktionsdauer richtet sich nach der Reaktivität der eingesetzten Ausgangsprodukte, nach der Wahl des jeweiligen Lösungsmittels, nach der Reaktionstemperatur und nach Art und Erscheinungsform der basischen Verbindung. Im allgemeinen liegt die Reaktionsdauer zwischen 2 und 20 Stunden.

Die Reaktionstemperatur ist vorzugsweise im Bereich zwischen 40 und 60 °C oder zwischen 60 und 80 °C, je nachdem, ob man ein Produkt mit einem besonders niedrigen Gehalt an hydrolysierbarem Halogen oder mit einem besonders hohen Epoxidierungsgrad erhalten möchten. Im allgemeinen erhält man bei tiefen Temperaturen Produkte, die sich durch einen besonders geringen Gehalt an hydrolysierbarem Halogen auszeichnen, während bei höheren Temperaturen ein Produkt mit einem höheren Epoxidierungsgrad anfällt.

Das Halogenhydrin wird bevorzugt in einer Menge von 1,0-20,0 Mol, bezogen auf eine phenolische Gruppe der phenolischen Komponente, eingesetzt. Besonders bevorzugt liegt das Halogenhydrin in Mengen von 1,5-10,0, ganz besonders 3,0-10,0, Mol pro phenolischer Hydroxylgruppe vor. Die Menge an basischer Verbindung wird vorzugsweise 50 gewählt, dass auf 1 Aequivalent phenolischer Hydroxylgruppe 1,0-10,0, bevorzugt 1,0-5,0, ganz besonders bevorzugt 1,0-3,0, Aequivalente Carbonatanionen entfallen. Die bevorzugte Lösungsmittelmenge beläuft sich auf 1,0 bis 20,0 Mol, bevorzugt auf 3,0 bis 10,0 Mol, pro Aequivalent phenolischer Gruppe.

In einer bevorzugten Ausführungsform des Verfahrens legt man die mindestens eine phenolische Hydroxylgruppe enthaltende Verbindung mit dem pulverisierten, wasserfreien Kaliumcarbonat im wasserfreien, aprotonischen, dipolaren Lösungsmittel vor, erhitzt die Lösung oder die Suspension auf 40-80 °C und gibt das Halogenhydrin unter Rühren zum Reaktionsgemisch. Die Zugabegeschwindigkeit wird dabei so eingestellt, dass die Reaktionstemperatur 80 °C nicht übersteigt. Falls erforderlich, lässt man anschliessend noch zwischen 40-80 °C nachreagieren. Die jeweils benötigte Reaktionsdauer kann anhand des Epoxidgehaltes der Reaktionsmischung verfolgt werden. Das Molverhältnis von phenolischer Komponente, Halogenhydrin und Lösungsmittel liegt bei dieser Ausführungsform zwischen 1:3:3 und 1:5:5. Pro Aequivalent phenolischer Gruppe werden 1,0-3,0 Mol basischer Verbindung zugesetzt.

Zur Aufarbeitung werden die festen Bestandteile vom Reaktionsgemisch abgetrennt, beispielsweise durch Filtration. Der Rückstand wird dann eingeengt und in einem mit Wasser nicht mischbaren organischen Lösungsmittel, beispielsweise in Methylethylketon oder Methylisobutylketon, in aromatischen Kohlenwasserstoffen, wie Benzol, Toluol oder Xylol, oder auch in aliphatischen Kohlenwasser-Stoffen, wie Hexan, Heptan oder Octan, aufgenommen. Diese Lösung wird dann solange mit Wasser gewaschen, bis sich der Gehalt an ionischen Verunreinigungen nicht mehr verringert. Anschliessend wird getrocknet, beispielsweise über $Na_2SO_4$, und das Lösungsmittel abgetrennt.

Setzt man Phenol- oder Kresol-Novolake als Ausgangsprodukte im erfindungsgemässen Verfahren ein, so ist es als besonders vorteilhaft anzusehen, dass sich bei den glycidylierten Endprodukten keine wesentliche Verschiebung in der Molekulargewichtsverteilung ergibt. Bei vorbekannten Glycidylierungsverfahren erfolgt in der Regel eine Erhöhung des Durchschnittsmolekulargewichts. Dies hat eine erhöhte Viskosität der Endprodukte zur Folge. Die erfindungsgemäss erhaltenen Glycidylierungsprodukte von Novolaken zeichnen sich durch eine überraschend niedrige Viskosität aus.

Die erfindungsgemäss erhältlichen Harze eignen sich insbesondere für den Einsatz auf den Gebieten des Oberflächenschutzes, der Elektrotechnik und Elektronik, der Laminierverfahren und im Bauwesen. Niedermolekulare Typen lassen sich als reaktive Verdünner einsetzen.

Die Harze können in jeweils dem speziellen Anwendungszweck angepasster Formulierung, in ungefülltem oder gefülltem Zustand als Komponente von Pressmassen, Wirbelsinterpulvern, Giessharzen, Spritzgussformulierungen, Imprägnierharzen, Klebmitteln, Werkzeugharzen, Laminierharzen und Pulverlacken eingesetzt werden.

Insbesondere eignen sich die erfindungsgemäss erhältlichen Glycidylierungsprodukte von Novolaken als Umhüllungsharze für elektronische Komponenten.

## Beispiele:

### Allgemeine Arbeitsvorschrift

In einem 1,5 l Sulfierkolben mit Kühler, Rührer, Thermometer und Stickstoffeinleitungsrohr werden 1 Aequivalent einer phenolischen Hydroxylgruppe enthaltenden Verbindung in ca. 5 Mol eines aprotonischen, dipolaren Lösungsmittels gelöst, mit 576 g (2 Mol) wasserfreiem, pulverisiertem Kaliumcarbonat bzw. 2 Mol Cäsiumcarbonat und 462,5 g (5,0 Mol) Epichlorhydrin versetzt und während 4-20 Stunden bei 40-80 °C zur Reaktion gebracht. Nach Beendigung der Reaktion wird das Gemisch auf Raumtemperatur abkühlen gelassen, filtriert und das Filtrat am Rotationsverdampfer bei 100-130 °C unter Vakuum eingeengt.

Der Rückstand wird in Methylisobutylketon gelöst, zweimal mit warmem Wasser gewaschen, die organische Phase über $Na_2S0_4$ getrocknet, filtriert und eingeengt.

Der Epoxydgehalt des entstehenden Rohproduktes liegt zwischen 70 und 97 % d.Th. Die Ergebnisse sind in der folgenden Tabelle dargestellt.

Die Molekulargewichte der polymeren Produkte werden mittels Gelpermeationschromatographie bestimmt. Die Reaktionsbedingungen sind in der folgenden Tabelle aufgeführt.

| Beispiel | Phenol | | Epi. Mol | Lösungsmittel | | Base | | Zeit [h] | Temp. [°C] | Ausbeute [%] | Epoxidgehalt | Molekulargewicht | |
|---|---|---|---|---|---|---|---|---|---|---|---|---|---|
| | Typ | Aeq.OH | | Typ | Mol | Typ | Mol | | | | [Aeq.Å/kg] | $\bar{M}_n$ | $\bar{M}_w$ |
| 1 | 2,6-Dimethylphenol | 1 | 5 | DMF | 5 | $K_2CO_3$ | 2 | 4 | 60 | 93 | 5,44 | | |
| 2 | 2,6-Dimethylphenol | 1 | 5 | DMSO | 5 | $K_2CO_3$ | 2 | 5 | 60 | 93 | 5,44 | | |
| 3 | p-Kresol | 1 | 5 | DMF | 5 | $K_2CO_3$ | 2 | 5 | 60 | 89 | 5,89 | | |
| 4 | Polyphenol-A 1) | 1 | 5 | DMF | 5 | $K_2CO_3$ | 2 | 5 | 60 | 89 | 4,80 | | |
| 5 | Phenol-Novolak 3) | 1 | 5 | DMSO | 5 | $K_2CO_3$ | 2 | 5 | 80 | 90,3 | 5,92 | | |
| 6 | Kresol-Novolak 2) | 1 | 5 | DMSO | 5 | $K_2CO_3$ | 2 | 20 | 40 | 93,1 | 5,14 | 920 | 1768 |
| 7 | Kresol-Novolak 2) | 1 | 5 | DMSO | 5 | $K_2CO_3$ | 2 | 5 | 60 | 89,6 | 5,41 | 788 | 1414 |
| 8 | Kresol-Novolak 2) | 1 | 5 | DMSO | 5 | $K_2CO_3$ | 2 | 5 | 80 | 96,5 | 5,21 | | |
| 9 | Kresol-Novolak 2) | 1 | 3 | DMSO | 3 | $K_2CO_3$ | 2 | 5 | 80 | 87,9 | 5,24 | | |
| 10 | Kresol-Novolak 2) | 1 | 5 | DMF | 5 | $K_2CO_3$ | 2 | 5 | 80 | 98,0 | 4,93 | | |
| 11 | Kresol-Novolak 2) | 1 | 5 | DMSO | 5 | $K_2CO_3$ | 2 | 10 | 50 | 91,0 | 5,16 | 931 | 1613 |

1) 1,1, 2,2'-Tetrakis-(4-hydroxyphenyl)-ethan

2) $\bar{M}_n$:701 $\bar{M}_w$:945

3) $\bar{M}_n$:401 $M_w$:518

| Beispiel | Phenol | | Epi. | Lösungsmittel | | Base | | Zeit [h] | Temp. [°C] | Ausbeute [%] | Epoxidgehalt | Molekulargewicht | | Chlorgehalt 4) |
|---|---|---|---|---|---|---|---|---|---|---|---|---|---|---|
| | Typ | Aeq.OH | | Typ | Mol | Typ | Mol | | | | [Aeq.Å/kg] | $\bar{M}_n$ | $\bar{M}_w$ | [ppm] |
| 12 | Kresol-Novolak 1) | 1 | 5 | DMSO | 5 | $K_2CO_3$ | 2 | 20 | 40 | 93 | 5,14 | 920 | 1768 | 213 |
| 13 | Kresol-Novolak 1) | 1 | 5 | $CH_3$-CN | 5 | $K_2CO_3$ 3) | 2 | 5 | 60 | 99 | 4,34 2) | | | |
| 14 | Kresol-Novolak 1) | 1 | 5 | DMSO | 5 | $K_2CO_3$ 3) | 2 | 10 | 40 | 100 | 5,04 | 993 | 2042 | 662 |
| 15 | Kresol-Novolak 1) | 1 | 5 | DMSO | 5 | $K_2CO_3$ 3) | 2 | 10 | 50 | 98 | 5,16 | 931 | 1613 | 730 |
| 16 | Kresol-Novolak 1) | 1 | 5 | $CH_3$-CN | 5 | $K_2CO_3$ | 2 | 5 | 60 | 95 | 3,84 2) | | | |
| 17 | Kresol-Novolak 1) | 1 | 5 | Sulfolan | 5 | $K_2CO_3$ | 2 | 5 | 60 | 84 | 4,71 | 1025 | | |
| 18 | Kresol-Novolak 1) | 1 | 5 | Propylencarbonat | 5 | $K_2CO_3$ | 2 | 5 | 60 | 95 | 3,75 2) | | 2052 | 2500 |
| 19 | Phenol | 1 | 5 | DMSO | 5 | $Cs_2CO_3$ | 2 | 5 | 60 | 92 | 6,19 | | | |
| 20 | 2,2-Bis-(4-hydroxyphenyl)-propan | 1 | 5 | DMSO | 5 | $K_2CO_3$ | 2 | 5,7 | 60 | 87,7 | 5,64 5) | | | |

1) $\bar{M}_n$ : 701 $\bar{M}_w$ : 945

2) Aufarbeitung nur bis zum Einengen mittels Rotationsverdampfer

3) zusätzlich mit 2 Mol% Tetrabutylammoniumhydrogensulfat (bezogen auf Gesamtgemisch)

4) hydrolysierbares Chlor

5) Nach dem Aufarbeiten mit Methylisobutylketon (Ausbeute: 95,6 %) erhält man ein Produkt mit einem Epoxidgehalt von 5,73 Aequ. Å/kg.

## Patentansprüche

1. Verfahren zur Herstellung von Glycidylverbindungen durch Umsetzung einer mindestens eine phenolische Gruppe enthaltenden Verbindung mit mindestens der aequivalenten Menge, bezogen auf die

phenolische Gruppe, eines Halogenhydrins in einem im wesentlichen wasserfreien, aprotonischen Lösungsmittel in Gegenwart einer festen, im wesentlichen wasserfreien, basischen Verbindung, dadurch gekennzeichnet, dass
die Umsetzung bei einer Temperatur zwischen 40 und 80 °C in Gegenwart von Alkalicarbonat erfolgt, wobei ein aprotonisches, dipolares Lösungsmittel mit einer statischen relativen Dielektrizitätskonstante von mehr als 25 (bei 25 °C) und einem permanenten elektrischen Dipolmoment von mehr als 2,5 D eingesetzt wird, das zusätzlich durch eine Uebergangsenergie der solvatochromen Absorptionsbande des gelösten N-(3,5-Diphenyl-4-hydroxyphenyl)-2,4,6-triphenylpyridiniumperchlorates zwischen 168 und 197,4 kJ/Mol (bei 25 °C) gekennzeichnet ist.

**2.** Verfahren gemäss Anspruch 1, worin als mindestens eine phenolische Gruppe enthaltende Verbindung Phenol, o-, m- oder p-Kresol, 2-Allylphenol, 2,6-Diallylphenol, 2,4,6-Triallylphenol, Bis-(Hydroxyphenyl)-methan, 1,1-Bis-(4-hydroxyphenyl)-ethan, 2,2-Bis-(4-hydroxyphenyl)-propan, Tetrabrom-Bisphenol A, Tetrachlor-Bisphenol A, 1,1-Bis-(4-hydroxyphenyl)-1-phenylethan, 1,1',2,2'-Tetrakis-(4-hydroxyphenyl)-ethan, Resorcin, Hydrochinon oder Polyphenole, die sich von der Kondensation von einkernigen Phenolen mit Aldehyden oder Ketonen ableiten (Novolake), eingesetzt werden.

**3.** Verfahren gemäss Anspruch 1, worin als Halogenhydrin Epichlorhydrin, β-Methylepichlorhydrin, Epibromhydrin, β-Methylepibromhydrin oder Glycerindichlorhydrin oder Gemische dieser Verbindungen eingesetzt werden.

**4.** Verfahren gemäss Anspruch 3, worin als Halogenhydrin Epichlorhydrin verwendet wird.

**5.** Verfahren gemäss Anspruch 1, worin als aprotonisches, dipolares Lösungsmittel 4-Methyl-1,3-dioxo-2-on, Acetonitril, Dimethylsulfoxid, Tetramethylensulfon, Dimethylformamid, Dimethylacetamid, 1-Methyl-2-pyrrolidon, Nitrobenzol oder Hexamethyl-phosphorsäuretriamid verwendet wird.

**6.** Verfahren gemäss Anspruch 5, worin Dimethylsulfoxid und/oder Dimethylformamid verwendet werden.

**7.** Verfahren gemäss Anspruch 1, worin als feste, im wesentlichen wasserfreie basische Verbindung Kaliumcarbonat oder Cäsiumcarbonat eingesetzt werden.

**8.** Verfahren gemäss Anspruch 7, worin Kaliumcarbonat verwendet wird.

**9.** Verfahren gemäss Anspruch 1, worin das Halogenhydrin in einer Menge von 1,0-20,0 Mol, bezogen auf eine phenolische Gruppe der phenolischen Komponente, eingesetzt wird.

**10.** Verfahren gemäss Anspruch 1, worin die Menge an basischer Verbindung so gewählt wird, dass auf 1 Aequivalent phenolischer Hydroxylgruppe 1,0-10,0 Aequivalente Carbonatanionen entfallen.

## Claims

**1.** A process for the preparation of a glycidyl compound by reacting a compound containing at least one phenolic group with at least the equivalent amount, based on the phenolic group, of a halohydrin in a substantially anhydrous, aprotonic solvent in the presence of a solid, substantially anhydrous, basic compound, which process comprises carrying out the reaction at a temperature between 40 and 80 °C in the presence of an alkali metal carbonate, an aprotonic dipolar solvent with a static relative dielectric constant of more than 25 (at 25 °C) and a permanent electric dipole moment of more than 2.5 D being used, an additional feature of the solvent being that the transition energy of the solvatochromic absorption band of the dissolved N-(3,5-diphenyl-4-hydroxyphenyl)-2,4,6-triphenylpyridinium perchlorate is between 168 and 197.4 kJ/mole (at 25 °C).

**2.** A process according to claim 1, wherein as the compound which contains at least one phenolic group phenol, o-, m- or p-cresol, 2-allylphenol, 2,6-diallylphenol, 2,4,6-triallylphenol, bis(hydroxyphenyl)-methane, 1,1-bis(4-hydroxyphenyl)ethane, 2,2-bis(4-hydroxyphenyl)propane, tetrabromo-bisphenol A, tetrachlorobisphenol A, 1,1-bis(4-hydroxyphenyl)-1-phenylethane, 1,1',2,2'-tetrakis(4-hydroxyphenyl)-ethane, resorcinol, hydroquinone or a polyphenol derived from condensation of a mononuclear phenol with an aldehyde or ketone (novolak) is used.

3. A process according to claim 1, wherein the halohydrin used is epichlorohydrin, β-methylepichlorohydrin, epibromohydrin, β-methylepibromohydrin or glycerol dichlorohydrin or a mixture of these compounds.

4. A process according to claim 3, wherein the halohydrin used is epichlorohydrin.

5. A process according to claim 1, wherein the aprotonic, dipolar solvent used is 4-methyl-1,3-dioxol-2-one, acetonitrile, dimethyl sulfoxide, tetramethylene sulfone, dimethylformamide, dimethylacetamide, 1-methyl-2-pyrrolidone, nitrobenzene or hexamethylphosphoric acid triamide.

6. A process according to claim 5, which comprises the use of dimethyl sulfoxide and/or dimethylformamide.

7. A process according to claim 1, wherein the solid, substantially anhydrous basic compound used is potassium carbonate or caesium carbonate.

8. A process according to claim 7, which comprises the use of potassium carbonate.

9. A process according to claim 1, which comprises using the halohydrin in an amount of 1.0-20.0 moles, based on one phenolic group of the phenolic component.

10. A process according to claim 1, wherein the amount of basic compound is chosen so that 1.0-10.0 equivalents of carbonate anions are present per equivalent of phenolic hydroxyl group.

## Revendications

1. Procédé de préparation de composés glycidyliques par réaction d'un composé contenant au moins un groupe phénolique avec une quantité au moins équivalente, par rapport aux groupes phénoliques, d'une halogénhydrine dans un solvant aprotique essentiellement anhydre, en présence d'un composé basique solide, essentiellement anhydre, caractérisé en ce que l'on effectue la réaction à une température comprise entre 40 et 80°C en présence d'un carbonate alcalin, en utilisant un solvant dipolaire aprotique ayant une constante diélectrique relative statique supérieure à 25 (à 25°C) et un moment dipolaire électrique permanent supérieur à 2,5 D, qui est caractérisé en outre par une énergie de transition de la bande d'absorption solvatochrome du perchlorate de N-(3,5-diphényl-4-hydroxyphényl)-2,4,6-tripnénylpyridinium dissous comprise entre 168 et 197,4 kJ/mole (à 25°C).

2. Procédé selon la revendication 1, dans lequel, comme composé contenant au moins un groupe phénolique, on utilise le phénol, l'o-, m- ou p-crésol, le 2-allylphénol, le 2,6-diallylphénol, le 2,4,6-triallylphénol, le bis(hydroxyphényl)méthane, le 1,1-bis(4-hydroxyphényl)éthane, le 2,2-bis(4-hydroxyphényl)propane, le tétrabromo-Bisphénol A, le tétrachloro-Bisphénol A, le 1,1-bis(4-hydroxyphényl)-1-phényléthane,le 1,1',2,2'-tétrakis(4-hydroxyphényl)éthane, le résorcinol, l'hydroquinone ou des polyphénols qui dérivent de la condensation de phénols monocycliques avec des aldéhydes ou des cétones (Novolaques).

3. Procédé selon la revendication 1, dans lequel on utilise comme halogénhydrine l'épichlorhydrine, la β-méthylépichlorhydrine,l'épibromhydrine, la β-méthylépibromhydrine ou la dichlorhydrine du glycérol, ou des mélanges de ces composés.

4. Procédé selon la revendication 3, dans lequel on utilise l'épichlorhydrine comme halogénhydrine.

5. Procédé selon la revendication 1, dans lequel, comme solvant dipolaire aprotique, on utilise la 4-méthyl-1,3-dioxo-2-one, l'acétonitrile, le diméthylsulfoxyde, la tétraméthylènesulfone, le diméthylformamide, le diméthylacétamide, la 1-méthyl-2-pyrrolidone, le nitrobenzène ou l'hexaméthylphosphotriamide.

6. Procédé selon la revendication 5, dans lequel on utilise le diméthylsulfoxyde et/ou le diméthylformamide.

7. Procédé selon la revendication 1, dans lequel, comme composé basique solide essentiellement anhydre, on utilise du carbonate de potassium ou du carbonate de césium.

8. Procédé selon la revendication 7, dans lequel on utilise du carbonate de potassium.

9. Procédé selon la revendication 1, dans lequel on utilise l'halogénhydrine en une quantité de 1,0-20,0 moles, rapportée à un groupe phénolique du constituant phénolique.

10. Procédé selon la revendication 1, dans lequel la quantité de composé basique est choisie de façon que, à 1 équivalent de groupe hydroxyle phénolique, correspondent 1,0-10,0 équivalents d'ions carbonate.